(19) Europäisches Patentamt
European Patent Office
Office européen des brevets

(11) Publication number: 0 336 326
A2

(12) # EUROPEAN PATENT APPLICATION

(21) Application number: 89105767.1

(22) Date of filing: 01.04.89

(51) Int. Cl.$^4$: C07K 5/06 , C07K 5/08 , C07K 1/00 , A61K 37/02

Claims for the following Contracting States: ES + GR.

(30) Priority: 07.04.88 IT 2011388

(43) Date of publication of application:
11.10.89 Bulletin 89/41

(84) Designated Contracting States:
AT BE CH DE ES FR GB GR IT LI LU NL SE

(71) Applicant: POLI INDUSTRIA CHIMICA S.p.A.
Piazza Agrippa, 1
I-20141 Milano(IT)

(72) Inventor: Poli, Stefano
Via Volturno, 48
I-20089 Quinto De' Stampi Rozzano Milan(IT)
Inventor: Coppi, Germano
Via Volturno, 48
I-20089 Quinto De' Stampi Rozzano Milan(IT)
Inventor: Del Corona, Lucio
Via Volturno, 48
I-20089 Quinto De' Stampi Rozzano Milan(IT)

(74) Representative: Minoja, Fabrizio
Studio Consulenza Brevettuale Via Rossini, 8
I-20122 Milano(IT)

(54) 3-Aminodihydrothiophene-2-one peptide derivatives, processes for the preparation and therapeutic use.

(57) Peptide derivatives of 3-aminodihydrothiophen-2-one having thiol and acylthiol moieties endowed with interesting mucolytic-expectorant, anti-emphysema, antibronchospastic, antitussive, anti-inflammatory, antioxidising (radical-scavengers), hepatoprotective and poorly toxic, are described.

The process for their preparation and the pharmaceutical formulations are also described.

EP 0 336 326 A2

# 3-AMINODIHYDROTHIOPHENE-2-ONE PEPTIDE DERIVATIVES, PROCESSES FOR THE PREPARATION AND THERAPEUTIC USE

The present invention relates to compounds of general formula I

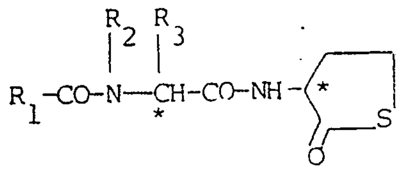

(I)

(* designating a chirality center)
wherein
$R_1$ is $C_1$-$C_4$ alkyl or a residue of formula

$R_4$-CH-
SR$_5$

$R_2$ is hydrogen or $C_1$-$C_4$ alkyl;
$R_3$ is hydrogen; $C_1$-$C_4$ alkyl; $C_1$-$C_4$ 1-thiolalkyl; or a residue of formula

$R_4$-CH-
SR$_6$

or
$R_2$ and $R_3$, taken together, are a $-CH_2-S-CH_2-$ chain;
$R_4$ is hydrogen or $C_1$-$C_4$ alkyl;
$R_5$ is hydrogen; $C_1$-$C_4$ alkylaminocarbonyl, $C_1$-$C_4$ alkanoyl, $C_4$-$C_8$ cycloalkylcarbonyl, aroyl, aralkanoyl, heterocyclocarbonyl having one or more S, N, O heteroatoms; and
$R_5$ can represent S bound to the same chain of formula I, to give a disulfide;
$R_6$ has the same meanings as $R_5$, with the proviso that only one between $R_5$ and $R_6$ can represent S bound to the same chain of formula I.

The present invention also relates to the single enantiomers and diastereoisomers, as well as to the respective mixtures thereof in any proportion, of compounds of formula I.

Moreover, the present invention also relates to possible pharmaceutically acceptable salts of compounds of formula I.

Among the compounds of formula I, particularly preferred are those in which $R_1$ is $C_1$-$C_2$ alkyl, or a residue of formula

$R_4$-CH- ;
SR$_5$

$R_2$ is hydrogen or $C_1$-$C_2$ alkyl;
$R_3$ is hydrogen, $C_1$-$C_2$ alkyl, 1-thiol-ethyl or a residue

$R_4$-CH- ;
SR$_6$

$R_2$ and $R_3$ together represent a $-CH_2-S-CH_2-$ chain;
$R_4$ is hydrogen or $C_1$-$C_2$ alkyl;
$R_5$ and $R_6$ are hydrogen, $C_1$-$C_4$ alkylaminocarbonyl; phenyl, acetyl, cyclopropylcarbonyl, cyclopentylcarbonyl, cyclohexylcarbonyl, benzoyl, phenylacetyl, 2-thenoyl, 3-thenoyl, 2-furoyl, imidazolylcarbonyl, nicotinoyl;
$R_5$ or $R_6$, moreover, represents S bound to the same chain of formula I to give a disulfide.

Compounds of formula I have interesting pharmacological properties; particularly, they have remarkable mucolytic and expectorant, antiemphysema, antibronchospastic, antitussive, antiinflammatory, antioxidant (radical-scavenger), hepatoprotective activities and, moreover, show an extremely poor toxicity.

More specifically, in the in vitro mucolytic activity test with a 4% mucin suspension (P. Pomarelli et al., Il Farmaco Ed. Prat., 33, 379, 1978) at equimolar concentrations (0.00625 M) after hydrolysis with rat serum, compounds II to VIII included (see Examples) respectively cause reductions in viscosity of 13.4%; 13.1%; 12.8%; 12.4; 13.4%; 13.6% and 13.2%; these reductions are significantly higher ($P < 0.01$) than those induced by tiopronin (11.5%) and by N-acetyl-cysteine (10.8%).

Furthermore, the compounds of the present invention have a remarkable mucolytic-expectorant activity, proved by means of the test of fluorescein elimination from the respiratory tract (G. Graziani, P. Cazzulani, Il

Farmaco, Ed. Prat., 36, 167, 1980). Compounds II to VIII included show, in comparison with the control ones, increase in the dye excretion of 72.0%; 71.3%; 68.5%; 67.6%; 74.1%; 71.9% and 70.8% respectively, which are significantly higher ($P < 0.01$) than those induced by tiopronin (55.8%) and N-acetylcysteine (45.6%), all of them being administered at 0.5 mM/kg doses orally.

The compounds of the present invention also display an interesting antiemphysema activity in the hamster (P.J. Stone et al., Amer. Rev. Respir. Dis., 124, 56, 1981). Emphysema induced by pancreatic elastase is morphometrically measured by evaluating, in µm, the mean linear intercept (MLI) (J. Kleinerman et al., Amer. Rev. Respir. Dis., 121, 381, 1980).

Compounds II to VIII included reduce the MLI increase in elastase-induced emphysema, by 30.6%; 32.7%; 31.8%; 32.7%; 35.6%; 34.9% and 35.4% respectively, in a statistically significant way ($P < 0.01$) after treatment at doses of 0.25 mM/kg per os, during 4 weeks.

The antibronchospastic activity of the compounds of the present invention was evaluated by the hystamine shock test in guinea pig (P. C. Braga et al., Min. Pneumol., 23, 447, 1984). Compounds II to VIII included increase resistance times against shock, compared with the basal values, respectively by 190.3%; 185.8%; 179.7%; 181.3%; 191.2%; 188.5% and 187.8%; said values being significantly higher ($P < 0.01$) than those of tiopronin (165.8%) and of N-acetylcysteine (167.5%), all the above compounds being administered at 0.25 mM/kg i.p. doses.

The antitussive activity of the compounds of the invention was evaluated by the citric acid induced cough test in the guinea pig (R. A. Turner, Screening methods in pharmacology, Acad. Press 1965, page 219). Compounds II to VIII included cause a reduction in cough strokes by 70.5%; 71.8%; 67.8%; 65.9%; 72.3%; 71.8% and 73.4% respectively, in comparison with the controls, at 0.25 mM/kg i.p. doses, said reduction being substantially comparable to that obtained with codeine phosphate (75.2%) at the dose of 5 mg/kg i.p.

The antiinflammatory activity was evaluated by the test of Bordetella pertussis induced edema in the rat (F. Capasso, Agents and Actions, 11, 741, 1981). Compounds II to VIII included reduce, in comparison with controls, the edema after 24 hours by 42.1%; 41.8%; 36.7%; 35.8%; 38.7%; 37.9% and 38.1% respectively, which values are significantly higher ($P < 0.01$) than those of tiopronin (28.6%) and of N-acetylcysteine (23.2%), all of said compounds being administered at doses of 0.5 mM/kg orally.

The antioxidant activity (radical-scavenger) was determined by evaluation of mortality in the rat after injection of E. coli 026B6 endotoxin (K. McKechnie et al., Circulat. Shock, 19, 429, 1986). Compounds II to VIII included, compared with a 100% mortality in the controls, cause survivals respectively of 55%; 50%; 45%; 40%; 55% and 60%, which are not significantly different from those obtained with DL-α-tocopherol, all the compounds being administered at doses of 0.25 mM/kg i.p.

The hepatoprotective activity was determined by the $CCl_4$intoxication test in the rat (E. C. Ferreyra et al., Toxic. Appl. Pharmacol., 27, 558, 1974). Compounds II to VIII included reduce serum GPT, in comparison with the intoxicated controls, by 52.3%; 54.1%; 48.7%; 47.6%; 52.8%; 51.9% and 54.1% respectively, which values are significantly higher ($P <0.01$) than the reductions induced by tiopronin (39.8%) and N-acetyl-cysteine (37.8%), all said compounds being administered in 0.25 mM/kg i.p. doses.

Furthermore, compounds II to VIII included reduce serum GOT, in comparison with intoxicated controls, by 55.8%; 54.7%; 52.7%; 56.1%; 58.5%; 59.3% and 60.1%, which reductions are significantly higher ($P < 0.01$) than those caused by tiopronin (42.2%) and by N-acetyl-cysteine (39.9%), all the compounds being administered at doses of 0.25 mM/kg i.p.

The compounds of the present invention show an extremely lox toxicity; $DL_{50}$ by the oral route for the tested compounds being always > 2.000 mg/kg.

Compounds of formula I and the possible pharmaceutically acceptable salts thereof, due to their pharmacological properties and low toxicity, can be used as drugs for the treatment of respiratory tract diseases and also as hepatoprotective agents.

Thus, according to another object, the present invention also relates to pharmaceutical compositions containing as the active ingredients the compounds of formula I (or the enantiomers and/or diastereoisomers thereof and/or their mixtures) or the pharmaceutically acceptable salts thereof for aerosol, parenteral, oral and rectal administrations in form of vials, oral suspensions, capsules, tablets, sachets or suppositories.

Examples of pharmaceutical formulations according to the invention are provided by:

- vials containing 50 to 300 mg of the active principle,
- syrup containing 100 to 600 mg of the active principle per dose,
- hard gelatin or Scherer capsules containing 100 to 600 mg of the active principle,
- sachets containing 100 to 600 mg of the active principle in a suitable excipient,
- suppositories containing 50 to 600 mg of the active principle in a suitable carrier for the neonatologic,

pediatric and adult uses.

The daily dose will range from 100 to 2.000 mg of the active principle in the treatment of respiratory tract diseases (particularly emphysema, asthma, chronic bronchitis, tracheobronchitis, etc.) and hepatic diseases (acute, infective, chronic hepatitis , hepatic steatosis, etc.).

Compounds I can be prepared by means of any one of the conventional procedures reported in chemistry and patent literature. For example, by reacting the metal salt, preferably the sodium or potassium salt, of a thiolacid with a 3-[(2-haloalkanoyl)glycyl]aminodihydrothiophene-2-one (a) in protic solvents such as water, alcohols, mixtures thereof, or in aprotic solvents such as dimethylformamide, dimethylsulfoxide, etc., at temperatures from 0° C to 100° C, preferably at 20° C, according to scheme A:

SCHEME A

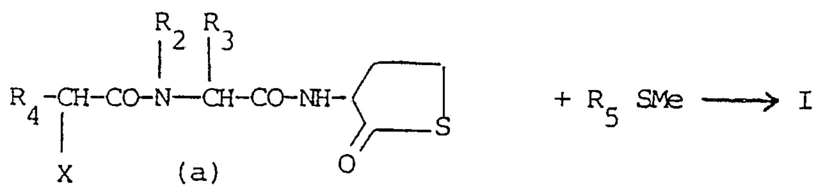

wherein X is a halogen, preferably chlorine or bromine, and Me is the cation or the equivalent of a metal, preferably sodium or potassium, while $R_2$, $R_3$, $R_4$ and $R_5$ have the above precised meanings; or by reacting halides of formula $R_5$-X with 3-[(2-thiolalkanoyl)glycyl]-aminodihydrothiophene-2-one metal salts under similar conditions, according to scheme B:

SCHEME B

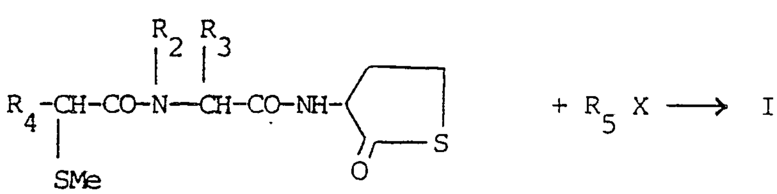

wherein $R_2$, $R_3$, $R_4$, $R_5$, Me and X have the above mentioned meanings; or by reacting acids of formula (b) with 3-aminodihydrothiophene-2-one in the presence of dicyclohexylcarbodiimide in inert solvents, or similarly reactive derivatives from said acids, such as halides, mixed anhydrides, esters, imidazolides, etc. with the same 3-aminodihydrothiophene-2-one according, to scheme C:

SCHEME C

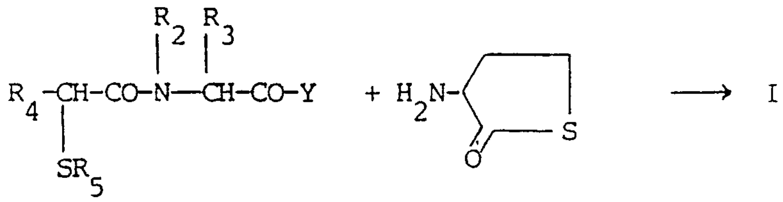

(b)

wherein $R_2$, $R_3$, $R_4$ and $R_5$ have the above mentioned meanings, while Y can be OH, Cl, Br, $COOC_2H_5$, $C_6Cl_5O$, 2.3.5-$C_6H_2Cl_3O$, N-imidazolyl, etc. or still, analogously, by reacting 2-acylthioalkanoic acids or the reactive derivatives thereof with 3-glycylaminodihydrothiophene-2-ones, according to scheme D:

SCHEME D

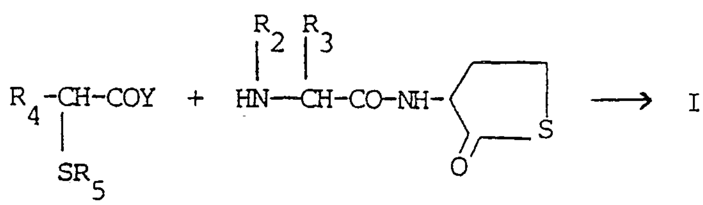

wherein $R_2$, $R_3$, $R_4$ and $R_5$ have the above mentioned meanings, while Y is an activating atom or group. Or, finally, by reducing 2,2′-disulfides of 3-(alkanoyl-glycyl)aminodihydrothiophene-2-ones (obtained in their turn from the corresponding disulfides according to the schemes C and D) by means of zinc in acid medium, according to scheme E:

SCHEME E

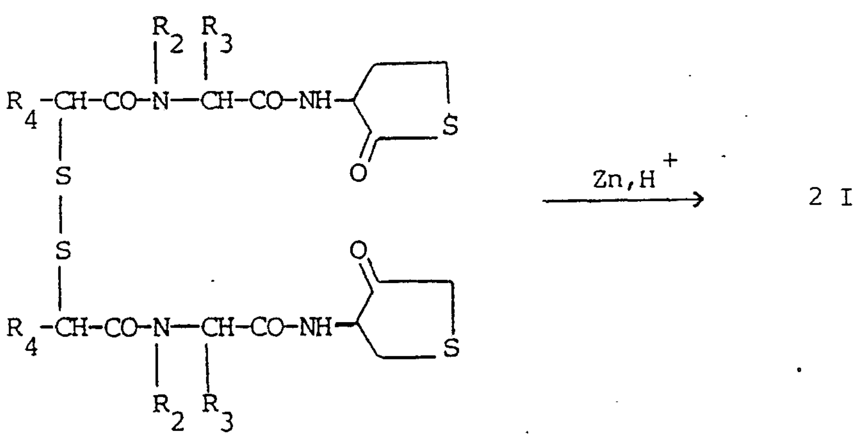


wherein $R_2$, $R_3$ and $R_4$ have the above mentioned meanings.

As already stated, the presence of asymmetry centers in formula I causes the existence of all the

possible stereoisomers, which also are an object of the present invention and which can be isolated or enriched according to conventional stereochemical procedures.

The present invention is illustrated in more detail by the following non-limiting Examples.

## Example 1 (Compound II)

A solution of 0.79 g (0.011 mole) of KHS in 10 ml of ethanol was added to a solution of 3.0 g (0.01 mole) of 3-[(2-bromopropionyl)glycyl]-aminodihydrothiophene-2-one in 25 ml of dimethylformamide. The reaction mixture was stirred for 120 minutes at 30°C, then filtered, the solvent was evaporated under vacuum, the residue was triturated with ethyl acetate till solidification, then recrystallized from aqueous isopropanol, to obtain 1.5 g (57%) of 3-[(2-thiolpropionyl)glycyl]aminodihydrothiophene-2-one.
Elemental analysis for $C_9H_{14}N_2O_3S_2$ (262):

| | | | | |
|---|---|---|---|---|
| Calc.: | C = 41.22% | H = 5.38% | N = 10.68% | S = 24.42% |
| Found: | C = 41.35% | H = 5.34% | N = 10.49% | S = 24.56% |

IR Spectrum: in accordance with the proposed structure.

## Example 2 (Compound III)

1.66 g (0.01 mole) of sodium thiophene-2-carboxylate was added under stirring, at 20°C, to a solution of 3.08 g (0.01 mole) of DL-3[2-bromopropionyl)glycyl]-aminodihydrothiophene-2-one in 25 ml of 80% ethanol. After 60 minute stirring at 20°C the reaction mixture was concentrated under vacuum at 30°C, the residue was taken up with water, filtered and the product was recrystallized from aqueous acetone, yielding 2.8 g (75%) of DL-3-[(2-thenoylthio)propionylglycyl]aminodihydrothiophene-2-one, m.p. 143-145°C.
Elemental analysis for $C_{14}H_{16}N_2O_4S_3$ (372):

| | | | | |
|---|---|---|---|---|
| Calc.: | C = 45.16% | H = 4.30% | N = 7.52% | S = 25.79% |
| Found: | C = 45.02% | H = 4.33% | N = 7.48% | S = 25.66% |

IR Spectrum: in accordance with the proposed structure.

## Example 3 (Compound IV)

1.54 g (0.01 mole) of N,N′-carbonyldiimidazole was added to a solution of 1.63 g (0.01 mole)of L-N-acetylcysteine in 15 ml of dimethylformamide, under stirring. The reaction mixture was stirred at 20°C for 60 minutes until gas evolution was over, then a solution obtained dissolving 1.54 g (0.01 mole) of 3-aminodihydrothiophene-2-one hydrochloride in 20 ml of DMF, adding 1.01 g (0.01 mole) of triethylamine and filtering the precipitated triethylamine hydrochloride, was added thereto. The mixture was left at 20°C for 2 hours, then concentrated under 0.1 mm Hg. The residual oil was mixed with 1.2 ml of acetic acid and 20 ml of water, then eluted on a column containing 50 ml of IR-120 in the $H^+$ form.

The eluate was concentrated to dryness, the obtained residue was crystallized from aqueous acetone, to obtain 1.4 g (53%) of L-3-[(N-acetylcysteinyl)]aminodihydrothiophene-2-one.
Elemental analysis for $C_9H_{14}N_2O_3S_2$ (262):

| | | | | |
|---|---|---|---|---|
| Calc.: | C = 41.22% | H = 5.38% | N = 10.68% | S = 24.42% |
| Found: | C = 41.28% | H = 5.35% | N = 10.60% | S = 24.51% |

IR Spectrum: in accordance with the proposed structure.

Example 4 (Compound V)

2.62 g of the product obtained in Example 3 were dissolved in 20 ml of DMF, 1.01 g (0.01 mole) of triethylamine was added, then a solution of 1.465 g (0.01 mole) of thiophene-2-carbonyl chloride dissolved in 10 ml of acetone was dropped at 0 °C, under stirring. The mixture was stirred for 60 minutes, the precipitated triethylamine hydrochloride was filtered, the filtrate was evaporated under 0.1 mm Hg and the residue was recrystallized from aqueous acetone, to obtain 2.85 g (76.6%) of 3-[N-acetyl-S-(2-thenoyl)-cysteinyl]aminodihydrothiophene-2-one. Elemental analysis for $CH_{14}H_{16}N_2O_4S_3$ (372):

| | | | | |
|---|---|---|---|---|
| Calc.: | C = 45.16% | H = 4.33% | N = 7.52% | S = 25.80% |
| Found: | C = 45.03% | H = 4.37% | N = 7.54% | S = 25.66% |

IR Spectrum: in accordance with the proposed structure.

Example 5 (Compound VI)

To a solution of 3.49 g (0.01 mole) of L-3-[(2-ethylaminocarbonylthiopropionyl)glycyl]thiazolidin-4-carboxylic acid in 20 ml of DMF, at 20 °C under stirring, 1.17 g (0.01 mole) of DL-3-aminodihydrothiophene-2-one and a solution of 2.2 g (0.011 mole) of dicyclohexylcarbodiimide in 10 ml of dimethylformamide were added. After 6 hours at room temperature dicyclohexylurea was filtered off, solvent was evaporated under 0.1 mm Hg and the residue was triturated with water to obtain 3.25 g (72.5%) of DL-3-{L-3-[(2-ethylaminocarbonylthiopropionyl)glycyl]thiazolidin-4-carbonyl}aminodihydrothiophene-2-one.

This compound was dissolved in 10 ml of ethanol, 20 ml of 1N sodium hydroxide were added to the solution, which was kept at 0 °C for 15 minutes. The mixture was acidified with hydrochloric acid to pH 2, the solvent was evaporated under vacuum, the residue was crystallized from aqueous alcohol to obtain 2.3 g (81.5%) of 3-{L-3-[(2-thiolpropionyl)glycyl]thiazolidin-4-carbonyl} aminodihydrothiophene-2-one. Elemental analysis for $C_{13}H_{19}N_3O_4S_3$ (377):

| | | | | |
|---|---|---|---|---|
| Calc.: | C = 41.37% | H = 5.08% | N = 11.14% | S = 25.46% |
| Found: | C = 41.12% | H = 5.11% | N = 11.07% | S = 25.39% |

IR Spectrum: in accordance with the proposed structure.

Example 6 (Compound VIII)

The solution obtained by dissolving 3.49 g (0.01 mole) of L-3-[(2-ethylaminocarbonylthiopropionyl)-glycyl]thiazolidin-4-carboxylic acid in 30 ml of 1N sodium hydroxide was acidified after 45 minutes with hydrochloric acid 1:1. The crystalline precipitate consisting of L-3-[(2-thiolpropionyl)glycyl]thiazolidin-4-carboxylic acid (Compound VII) was filtered and dried to give 2.05 g (73.7%); m.p. 182-184 °C. Elemental analysis for $C_9H_{14}N_2O_4S_2$(278):

| | | | | |
|---|---|---|---|---|
| Calc.: | C = 38.84% | H 5.07% | N = 10.07% | S = 23.02% |
| Found: | C = 38.94% | H 5.11% | N = 9.93% | S = 23.17% |

IR Spectrum: in accordance with the proposed structure.

This compound was dissolved in 15 ml of 1N sodium hydroxide and 1.17 g (0.008 mole) of 2-thenoyl chloride was added to the ice-cooled solution, under strong stirring.

15 minutes after the solution was acidified with 1:1 hydrochloric acid, the precipitate was filtered and dried obtaining 1.93 g (67.7%) of L-3-[(2-thenoylthiopro pionyl)glycyl]-thiazolidin-4-carboxylic acid, which was dissolved in 15 ml of dimethylformamide.

0.8 g (0.005 mole) of N,N′-carbonyldiimidazole was added to the solution and, after 60 minutes stirring at room temperature, a solution of 0.59 g (0.005 mole) of 3-aminodihydrothiophene-2-one was added. The mixture was left for 4 hours at 25° C, the solvent was evaporated under 0.1 mm Hg, the residue was poured into ice and the precipitated product was filtered and recrystallized from aqueous acetone, to obtain 1.3 g (54%) of 3-{L-3-[(2-thenoylthiopropionyl)glycyl]thiazolidin-4-carbonyl}aminodihydrothiophene-2-one (compound VIII) m.p. 173-174° C.
Elemental analysis for $C_{18}H_{21}N_3O_5S_4$ (487):

| | | | | |
|---|---|---|---|---|
| Calc.: | C = 44.35% | H = 4.34% | N = 8.62% | S = 26.28% |
| Found: | C = 44.29% | H = 4.38% | N = 8.49% | S = 26.21% |

IR Spectrum: in accordance with the proposed structure.

## Claims

1. Compounds of general formula I

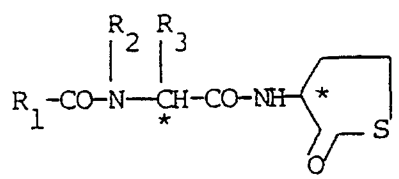

(I)

(* designating a chirality center)
wherein
$R_1$ is $C_1$-$C_4$ alkyl or a residue of formula
$R_4$-CH-
$SR_5$
$R_2$ is hydrogen or $C_1$-$C_4$ alkyl;
$R_3$ is hydrogen; $C_1$-$C_4$ alkyl; $C_1$-$C_4$ 1-thiolalkyl; or a residue of formula
$R_4$-CH-
$SR_6$
or
$R_2$ and $R_3$, taken together, are a $-CH_2-S-CH_2-$ chain;
$R_4$ is hydrogen or $C_1$-$C_4$ alkyl;
$R_5$ is hydrogen; $C_1$-$C_4$ alkylaminocarbonyl, $C_1$-$C_4$ alkanoyl, $C_4$-$C_8$ cycloalkylcarbonyl, aroyl, aralkanoyl, heterocyclocarbonyl having one or more S, N, O heteroatoms; and
$R_5$ can represent S bound to the same chain of formula I, to give a disulfide;
$R_6$ has the same meanings as $R_5$, with the proviso that only one between $R_5$ and $R_6$ can represent S bound to the same chain of formula I;
their enantiomers and diastereoisomers, as well as the respective mixtures thereof in any proportion and the pharmaceutically acceptable salts thereof.

2. Compounds as claimed in claim 1, wherein:
$R_1$ is $C_1$-$C_2$ alkyl, or a residue of formula
$R_4$-CH- ;
$SR_5$
$R_2$ is hydrogen or $C_1$-$C_2$ alkyl;
$R_3$ is hydrogen, $C_1$-$C_2$ alkyl, 1-thiol-ethyl or a residue
$R_4$-CH- ;
$SR_6$
$R_2$ and $R_3$ together represent a $-CH_2-S-CH_2-$ chain;
$R_4$ is hydrogen or $C_1$-$C_2$ alkyl;
$R_5$ and $R_6$ are hydrogen, $C_1$-$C_4$ alkylaminocarbonyl; phenyl, acetyl, cyclopropylcarbonyl, cyclopentylcarbonyl, cyclohexylcarbonyl, benzoyl, phenylacetytl, 2-thenoyl, 3-thenoyl, 2-furoyl, imidazolylcarbonyl, nicotinoyl; $R_5$ or $R_6$, moreover, represents S bound to the same chain of formula I to give a disulfide.

3. A compound as claimed in claims 1-2, selected from the group consisting of:
- 3-[(2-thiolpropionyl)glycyl]aminodihydrothiophene-2-one;
- 3-[(2-thenoylthio)propionylglycyl]aminodihydrothiophene-2-one;
- 3-(N-acetylcysteinyl)-aminodihydrothiophene-2-one;
- 3-[N-acetyl-S-(2-thenoyl)cysteinyl]-aminodihydrothiophene-2-one;
- 3-{L-3-[(2-thiolpropionyl)glycyl]thiazolidin-4-carbonyl} aminodihydrothiophene-2-one;
- 3-{L-3-[(2-thenoylthiopropionyl)glycyl]thiazolidin-4-carbonyl}aminodihydrothiophene-2-one.

4. A process for the preparation of the compounds as claimed in claim 1, characterized by the following steps:

a) reacting the metal salt, of a thiolacid with a 3-[(2-haloalkanoyl)glycyl]aminodihydrothiophene-2-one (a) in protic solvents such as water, alcohols, mixtures thereof, or in protic solvents such as dimethylformamide, dimethylsulfoxide, etc., at temperatures from 0°C to 100°C, preferably at 20°C, according to scheme A:

SCHEME A

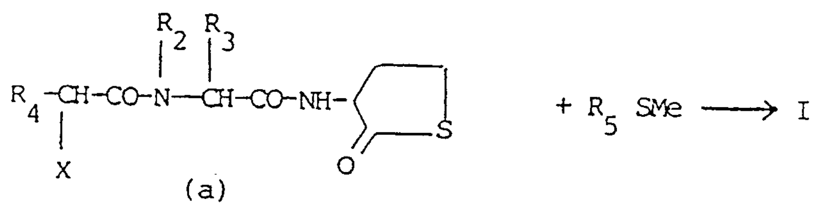

wherein X is a halogen, preferably chlorine or bromine, and Me is the cation or the equivalent of a metal, preferably sodium or potassium, while $R_2$, $R_3$, $R_4$ and $R_5$ have the above precised meanings; or

b) reacting halides of formula $R_5$-X with 3-[(2-thiolalkanoyl)glycyl]-aminodihydrothiophene-2-one metal salts under similar conditions, according to scheme B:

SCHEME B

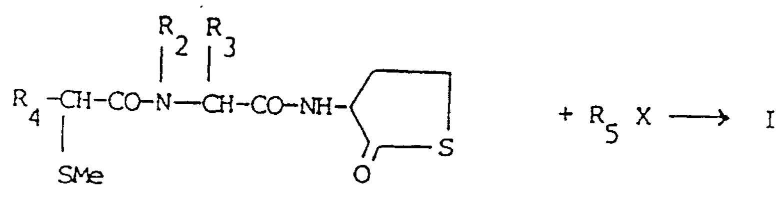

wherein $R_2$, $R_3$, $R_4$, $R_5$, Me and X have the above mentioned meanings; or

c) reacting acids of formula (b) with 3-aminodihydrothiophene-2-one in the presence of dicyclohexylcarbodiimide in inert solvents, or similarly reactive derivatives from said acids, such as halides, mixed anhydrides, esters, imidazolides, etc. with the same 3-aminodihydrothiophene-2-one according, to scheme C:

SCHEME C

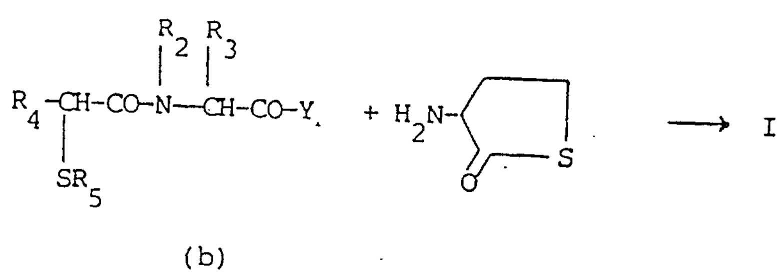

wherein $R_2$, $R_3$, $R_4$ and $R_5$ have the above mentioned meanings, while Y can be OH, Cl, Br, $COOC_2H_5$, $C_6Cl_5O$, 2.3.5-$C_6H_2Cl_3O$, N-imidazolyl, etc. or

d) reacting 2-acylthioalkanoic acids or the reactive derivatives thereof with 3-glycylaminodihydrothiophene-2-ones, according to scheme D:

SCHEME D

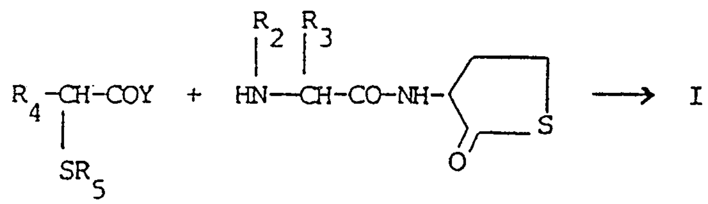

wherein $R_2$, $R_3$, $R_4$ and $R_5$ have the above mentioned meanings, while Y is an activating atom or group, or

e) reducing 2,2′-disulfides of 3-(alkanoyl-glycyl)aminodihydrothiophene-2-ones (obtained in their turn from the corresponding disulfides according to the schemes C and D) by means of zinc in acid medium, according to scheme E:

SCHEME E

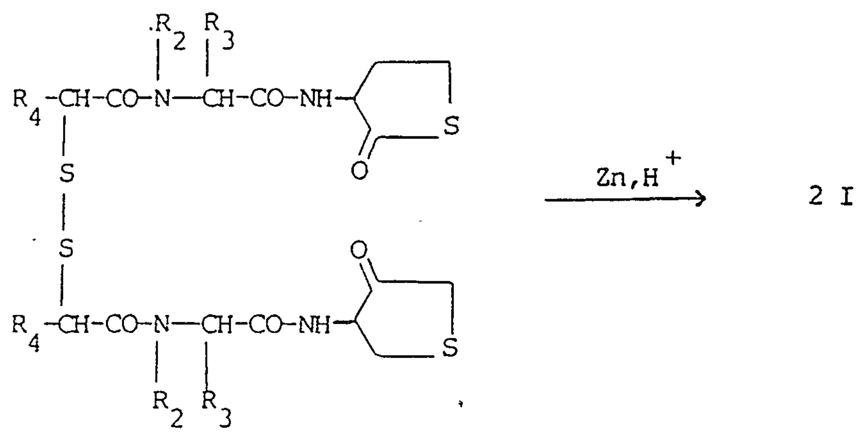


wherein $R_2$, $R_3$ and $R_4$ have the above mentioned meanings.

5. Pharmaceutical compositions containing as the active ingredient the compounds as claimed in claims 1-3, in admixture with a pharmaceutically acceptable carrier.

6. Pharmaceutical compositions as claimed in claim 5, in form of vials, oral suspensions, capsules, tablets, sachets or suppositories containing 50 to 600 mg of the active ingredient.

7. A compound as claimed in claims 1-3 for use as a therapeutic agent.

8. The use of a compound as claimed in claims 1-3 or of a salt thereof for the preparation of a pharmaceutical formulation for the the treatment of diseases of the respiratory tract, such as emphysema, asthma, chronic bronchitis, tracheobronchitis, and of hepatic diseases such as acute, infective, chronic hepatitis, hepatic steatosis, etc.

Claims for the following Contracting States: GR and ES

1. A process for the preparation of compounds of general formula I

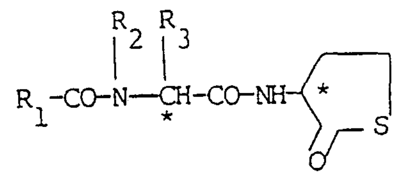

(I)

(* designating a chirality center)
wherein
$R_1$ is $C_1$-$C_4$ alkyl or a residue of formula
$R_4$-CH-
   S$R_5$
$R_2$ is hydrogen or $C_1$-$C_4$ alkyl;
$R_3$ is hydrogen; $C_1$-$C_4$ alkyl; $C_1$-$C_4$ 1-thiolalkyl; or a residue of formula
$R_4$-CH-
   S$R_6$
or
$R_2$ and $R_3$, taken together, are a $-CH_2$-S-$CH_2$- chain;
$R_4$ is hydrogen or $C_1$-$C_4$ alkyl;
$R_5$ is hydrogen; $C_1$-$C_4$ alkylaminocarbonyl, $C_1$-$C_4$ alkanoyl, $C_4$-$C_8$ cycloalkylcarbonyl, aroyl, aralkanoyl, heterocyclocarbonyl having one or more S, N, O heteroatoms; and
$R_5$ can represent S bound to the same chain of formula I, to give a disulfide;
$R_6$ has the same meanings as $R_5$, with the proviso that only one between $R_5$ and $R_6$ can represent S bound to the same chain of formula I;
their enantiomers and diastereoisomers, as well as the respective mixtures thereof in any proportion and the pharmaceutically acceptable salts thereof, which process is characterized by the following steps:

(a) reacting the metal salt, of a thiolacid with a 3-[(2-haloalkanoyl)glycyl]aminodihydrothiophene-2-one (a) in protic solvents such as water, alcohols, mixtures thereof, or in protic solvents such as dimethylformamide, dimethylsulfoxide, etc., at temperatures from 0°C to 100°C, preferably at 20°C, according to scheme A:

SCHEME A

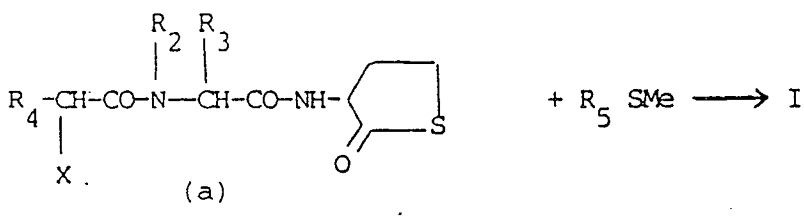

(a)

wherein X is a halogen, preferably chlorine or bromine, and Me is the cation or the equivalent of a metal, preferably sodium or potassium, while $R_2$, $R_3$, $R_4$ and $R_5$ have the above precised meanings; or

b) reacting halides of formula $R_5$-X with 3-[(2-thiolalkanoyl)glycyl]-aminodihydrothiophene-2-one metal salts under similar conditions, accoridng to scheme B:

SCHEME B

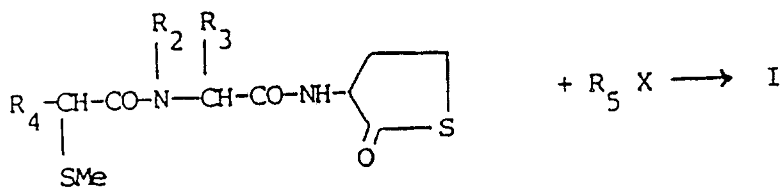

wherein $R_2$, $R_3$, $R_4$, $R_5$, Me and X have the above mentioned meanings; or

c) reacting acids of formula (b) with 3-aminodihydrothiophene-2-one in the presence of dicyclohexylcarbodiimide in inert solvents, or similarly reactive derivatives from said acids, such as halides, mixed anhydrides, esters, imidazolides, etc. with the same 3-aminodihydrothiophene-2-one according, to scheme C:

SCHEME C

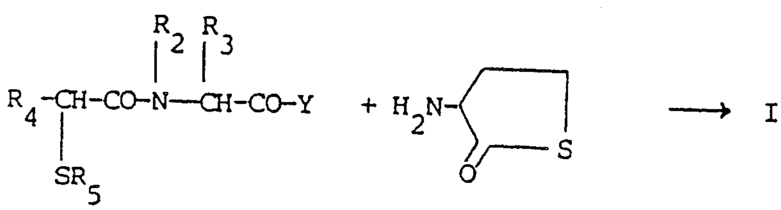

(b)

wherein $R_2$, $R_3$, $R_4$ and $R_5$ have the above mentioned meanings, while Y can be OH, Cl, Br, $COOC_2H_5$, $C_6Cl_5O$, 2.3.5-$C_6H_2Cl_3O$, N-imidazolyl, etc. or

d) reacting 2-acylthioalkanoic acids or the reactive deri vatives thereof with 3-glycylaminodihydrothiophene-2-ones, according to scheme D:

SCHEME D

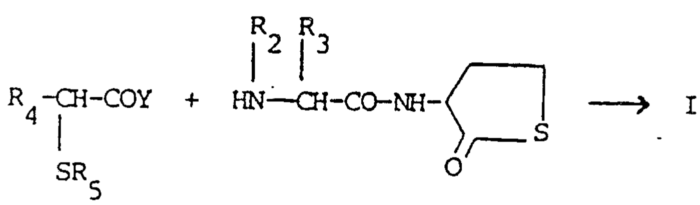

wherein $R_2$, $R_3$, $R_4$ and $R_5$ have the above mentioned meanings, while Y is an activating atom or group, or

e) reducing 2,2′-disulfides of 3-(alkanoyl-glycyl)aminodihydrothiophene-2-ones (obtained in their turn from the corresponding disulfides according to the schemes C and D) by means of zinc in acid medium, according to scheme E:

SCHEME E

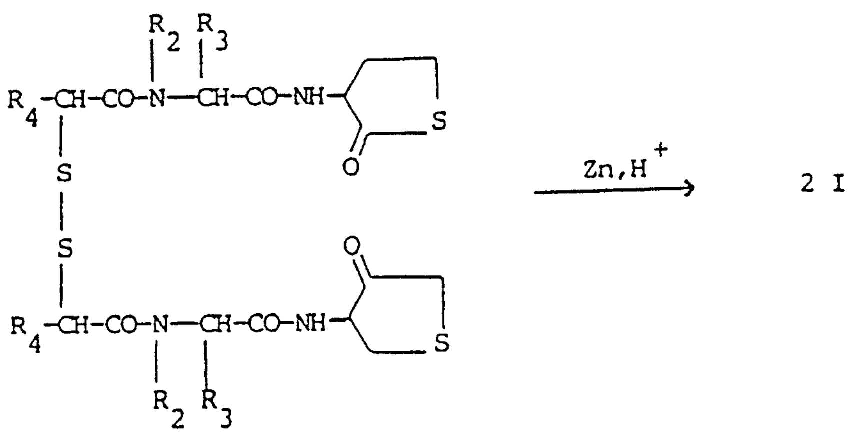


wherein $R_2$, $R_3$ and $R_4$ have the above mentioned meanings.